# FASCICULE DE BREVET EUROPEEN

(11) **EP 2 324 884 B1**
(45) Date de publication et mention de la délivrance du brevet: **02.01.2013**
(21) Numéro de dépôt: 10178574.9
(22) Date de dépôt: 23.09.2010
(51) Int. Cl.: A61N 1/362, A61N 1/365

(54) **Dispositif médical implantable actif de resynchronisation cardiaque avec optimisation de l'asservissement à l'effort de la fréquence de stimulation**
Aktives medizinisches Implantat zur Herzresynchronisierung mit Steuerungsoptimierung der Belastung der Stimulierungsfrequenz
Active implantable medical device for cardiac resynchronisation with optimisation of the closed-loop control under the force of the stimulation frequency

(30) Priorité: 23.11.2009 FR 0958279
(43) Date de publication de la demande: 25.05.2011
(73) Titulaire: Sorin CRM SAS, 92143 Clamart Cedex (FR)
(72) Inventeur: Limousin, Marcel, 75014 Paris (FR)
(74) Mandataire: Dupuis-Latour, Dominique

(56) Documents cités:
- EP-A1- 0 750 920
- EP-A1- 1 524 009
- WO-A1-92/03182
- US-A- 5 609 613

## Description

L'invention concerne les "dispositifs médicaux implantables actifs" tels que définis par la directive 90/385/CEE du 20 juin 1990 du Conseil des communautés européennes.

Elle concerne plus précisément les implants permettant de surveiller en continu le rythme cardiaque et délivrer si nécessaire au coeur des impulsions électriques permettant d'assurer une stimulation conjointe et permanente des ventricules gauche et droit afin de resynchroniser ces derniers, technique dite "CRT" *(Cardiac Resynchronization Therapy)* ou "BVP" (*Bi-Ventricular Pacing).*

À cet effet, on implante au patient un appareil muni d'électrodes permettant de stimuler l'un et l'autre ventricule. Le dispositif est capable de surveiller en continu le rythme cardiaque et délivrer si nécessaire au coeur des impulsions électriques pour stimuler concurremment les ventricules gauche et droit afin de resynchroniser ces derniers, en appliquant entre les instants respectifs de stimulation un délai dit "délai interventriculaire" (DVV ou VVD) ajusté de manière à resynchroniser la contraction des deux ventricules pour optimiser l'état hémodynamique du patient.

Un tel stimulateur CRT est par exemple divulgué dans le EP 1 108 446 A1 (Sorin CRM, anciennement ELA Medical).

Comme tout stimulateur, un stimulateur CRT comprend des moyens pour asservir le rythme des impulsions de stimulation en fonction du niveau d'effort du patient, avec une fréquence moindre lorsque le patient est au repos et une fréquence progressivement croissante au fur et à mesure qu'augmente l'effort. Ce dernier paramètre est mesuré par un capteur approprié tel qu'un capteur physiologique (typiquement un capteur de ventilation-minute ou "capteur MV") et/ou un capteur d'activité physique (typiquement un accéléromètre ou "capteur G").

La fréquence de stimulation ainsi asservie peut varier entre une fréquence minimale dite "fréquence de base" (f_{base}) et une fréquence maximale (fₘₐₓ) qui définit un plafond pour la fréquence de stimulation calculée par les algorithmes d'asservissement.

On notera toutefois que la fréquence instantanée du patient peut être soit une fréquence spontanée (sinusale), soit une fréquence stimulée, et que ce n'est que dans ce dernier cas que la fréquence des impulsions de stimulation est gérée par le dispositif en fonction du niveau d'effort du patient.

Le EP 1 069 099 A1 (Sorin CRM, anciennement ELA Medical) décrit un stimulateur CRT pourvu de moyens permettant notamment d'ajuster sur le long terme le paramètre fₘₐₓ en fonction de l'évolution de l'état général du patient.

L'indication typique des stimulateurs CRT est l'insuffisance cardiaque, qui est une pathologie induisant une dilatation des cavités cardiaques, avec réduction significative de la fraction d'éjection et épuisement rapide en cas d'effort, même faible.

Chez un patient sain, lors d'un effort le coeur met en action deux mécanismes pour répondre aux besoins hémodynamiques, à savoir l'augmentation de la fréquence cardiaque (rythme sinusal spontané) et l'adaptation de la contractilité (qui a une incidence sur les volumes éjectés).

En revanche, en présence d'une insuffisance cardiaque, le patient ne présente que peu d'adaptabilité en ce qui concerne la contractilité du muscle cardiaque.

En outre, ces patients sont en général sous traitement médicamenteux intensif, avec notamment prise de bêta-bloquants pour ralentir la fréquence cardiaque de ces patients, dont la fréquence moyenne spontanée au repos est généralement plus élevée. Mais ces bêta-bloquants et autres traitements ont pour conséquence de réduire la capacité d'accélération du rythme cardiaque, donc une moindre réactivité à l'effort.

Il s'ensuit un épuisement rapide à l'effort modéré lorsque le patient est en classe II de la NYHA *(New York Heart Association),* voire même un épuisement rapide quel que soit le niveau d'exercice, c'est-à-dire même pour les actes très simples de la vie courante, chez des patients en classes III et IV.

La thérapie de resynchronisation CRT, par stimulation multisite, permet de pallier certaines conséquences de l'insuffisance cardiaque grâce à une meilleure synchronisation entre les deux ventricules, qui permet d'accroître la contractilité et d'augmenter la phase de remplissage.

En revanche, du fait de la prise de bêta-bloquants, lors d'un effort même peu soutenu le potentiel d'augmentation de la fréquence cardiaque est limité par l'effet chronotrope négatif de ces traitements.

Certes, les appareils multisite possèdent des asservissements à l'effort de la fréquence de stimulation.

Le WO 92/03182 A1 décrit un tel stimulateur *rate-responsive* simple-chambre conventionnel - c'est-à-dire non pourvu de moyens de resynchronisation CRT. L'asservissement de la fréquence de stimulation y est conçu pour être le plus physiologique possible, donc avec une variation très progressive et lente, de l'ordre de plusieurs dizaines de secondes ; l'exemple décrit par ce document prévoit ainsi une transition de la fréquence de base à la fréquence maximale en soixante secondes environ. Si l'on applique ces algorithmes d'asservissement conventionnels, destinés aux patients souffrant de bradycardie, à un patient souffrant d'insuffisance cardiaque, les effets produits seront inadaptés, voir délétères. En effet :
- d'une part, si au début de l'effort le patient est en rythme spontané avec une fréquence supérieure à la fréquence de stimulation calculée par l'algorithme d'asservissement, alors le patient ne sera pas stimulé : ceci induira un retard supplémentaire à l'adaptation, car le patient ne bénéficiera de l'asservissement de fréquence que lorsque cet asservissement calculera une fréquence de stimulation supérieure à la fréquence courante spontanée du patient ;
- d'autre part, le profil d'accroissement des asservissements de la fréquence de stimulation est conçu pour appliquer un accroissement lent et progressif de la fréquence, notamment au début de l'effort : dans ce cas, lorsque le patient commencera à être stimulé, la fréquence n'augmentera que lentement pour un effort modéré, avec une pente d'accélération plus importante seulement pour les efforts soutenus.

Un tel profil d'asservissement de fréquence est donc mal adapté à un patient souffrant d'insuffisance cardiaque, car ces patients n'effectuent en général que rarement des efforts soutenus. De ce fait, ils ne bénéficient pas d'une augmentation significative et rapide de la fréquence cardiaque, dont la dynamique d'augmentation est en outre limitée par l'effet chronotrope négatif des traitements médicamenteux qui leur sont administrés. L'invention part de cette constatation clinique. L'un des buts de l'invention est ainsi de proposer un dispositif de type CRT qui remédie aux divers inconvénients exposés ci-dessus, c'est-à-dire un dispositif CRT dont l'asservissement de la fréquence de stimulation présente un profil adapté aux insuffisants cardiaques, qui sont les patients pour lesquels ce type de dispositif est le plus indiqué.

Le dispositif de l'invention est un stimulateur CRT, par exemple tel que divulgué dans le EP 1 108 446 A1 ou EP 1 059 099 A1 précité, c'est-à-dire comprenant : des moyens pour déterminer une valeur courante de fréquence spontanée du patient en présence d'un rythme sinusal ; un capteur d'effort, délivrant un signal représentatif du niveau courant d'effort du patient ; des moyens pour détecter le début d'un effort à partir du signal délivré par le capteur ; des moyens pour calculer une fréquence de stimulation asservie à l'effort, cette fréquence de stimulation pouvant varier entre une fréquence de base et une fréquence maximale en fonction du signal délivré par le capteur ; et des moyens pour délivrer sélectivement des impulsions de stimulation à la fréquence de stimulation calculée.

De façon caractéristique de l'invention, les moyens CRT de stimulation synchronisée comprennent en outre des moyens d'incrémentation accélérée de la fréquence de stimulation aptes à exécuter des commandes de : (i) détermination d'une fréquence-cible fonction de l'écart entre d'une part la plus élevée d'entre la fréquence de base et, le cas échéant, la fréquence spontanée, et d'autre part la fréquence maximale ; et (ii) augmentation directe, sur détection d'un début d'effort, de la fréquence de stimulation depuis la valeur initiale courante de la fréquence de stimulation, ou de la fréquence spontanée, jusqu'à la fréquence-cible en un laps de temps prédéterminé, ou sur un nombre de cycles cardiaques prédéterminé, permettant d'atteindre ladite fréquence-cible en au plus quatre cycles cardiaques.

De préférence, le dispositif comprend en outre des moyens pour définir une pluralité de zones d'effort consécutives sur l'étendue de la dynamique du signal délivré par le capteur d'effort. Les moyens d'incrémentation accélérée de la fréquence de stimulation sont alors aptes à exécuter distinctement pour chaque zone lesdites commandes de détermination de fréquence-cible et d'augmentation directe de la fréquence de stimulation jusqu'à cette fréquence-cible, la fréquence-cible d'une zone étant fonction de l'écart entre, d'une part, la fréquence-cible de la zone immédiatement précédente et, d'autre part, la fréquence maximale.

Ces zones d'effort consécutives peuvent notamment être définies par une division égale de l'étendue de la dynamique du signal délivré par le capteur d'effort.

Il est possible de prévoir des moyens de mise à jour des valeurs extrêmes de la dynamique du signal délivré par le capteur d'effort, avec dans ce cas redéfinition de la pluralité de zones d'effort consécutives.

La fréquence-cible est de préférence déterminée par un pourcentage, par exemple 50%, de l'écart entre, d'une part, la plus élevée d'entre la fréquence de base et, le cas échéant, la fréquence spontanée, et, d'autre part, la fréquence maximale.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 représente de façon schématique la caractéristique donnant la fréquence de stimulation en fonction de l'information délivrée par le capteur d'effort, pour un stimulateur CRT de type connu et pour un stimulateur selon l'invention.
La Figure 2 illustre une autre caractéristique du même type, dans le cas d'une augmentation plus rapide de l'effort à partir d'une situation de rythme stimulé.
La Figure 3 est homologue de la Figure 2, à partir d'une situation de rythme spontané.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Ovatio CRT* ou *Paradym CRT* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. Ces dispositifs comportent divers moyens permettant d'appliquer une stimulation ventriculaire resynchronisée, avec des moyens pour asservir la fréquence de stimulation au niveau d'effort du patient, dans le cas où celui-ci n'est pas en rythme spontané.

Le dispositif dispose pour cela d'un ou plusieurs capteurs d'effort, qui peuvent être constitués d'un capteur physiologique et/ou d'un capteur d'activité.

Un capteur physiologique est un capteur censé donner une représentation adéquate des besoins métaboliques instantanés du patient à un instant donné, typiquement un capteur de ventilation-minute (capteur MV). Le capteur d'activité est un capteur permettant de détecter rapidement un changement de l'activité du porteur de l'appareil, typiquement un accéléromètre (capteur G). Le stimulateur peut être équipé de ces deux types de capteurs qui opèrent une "surveillance croisée" de leurs indications respectives, combinées entre elles pour donner une information représentative du niveau d'effort du patient, ci-après désignée "information du capteur d'effort", pour asservir de façon appropriée la fréquence cardiaque. Le capteur, ou la combinaison de capteurs, permettant de délivrer un tel signal sera désigné de façon générale "capteur d'effort", sans que cette dénomination soit restrictive d'un type particulier de capteur.

De façon générale, la fréquence cardiaque instantanée du patient est soit une fréquence spontanée (sinusale), soit une fréquence stimulée, en cas d'absence de rythme spontané ou bien si l'algorithme estime que le rythme spontané n'est pas compatible avec le niveau d'effort du patient. Dans ce dernier cas, la fréquence de stimulation est gérée par un asservissement du dispositif, qui fait varier la fréquence de stimulation entre une valeur minimale dite "fréquence de base" (f_{base}), par exemple 60 cpm, et une fréquence maximale fₘₐₓ, par exemple 120 cpm, ces deux bornes extrêmes étant paramétrées initialement par le médecin, puis mises à jour régulièrement par le dispositif au cours de phases d'auto-calibration exécutées périodiquement, selon des techniques en elles-mêmes connues et qui ne seront pas décrites plus en détail.

Une simulation à f_{base} correspond à la valeur minimale de l'information du capteur d'effort (une valeur nulle dans le cas d'un accéléromètre), et une stimulation à fₘₐₓ à la valeur la plus élevée susceptible d'être atteinte par cette même information, correspondant au maximum d'activité du capteur. L'écart entre ces deux informations extrêmes est appelé dynamique du capteur d'effort.

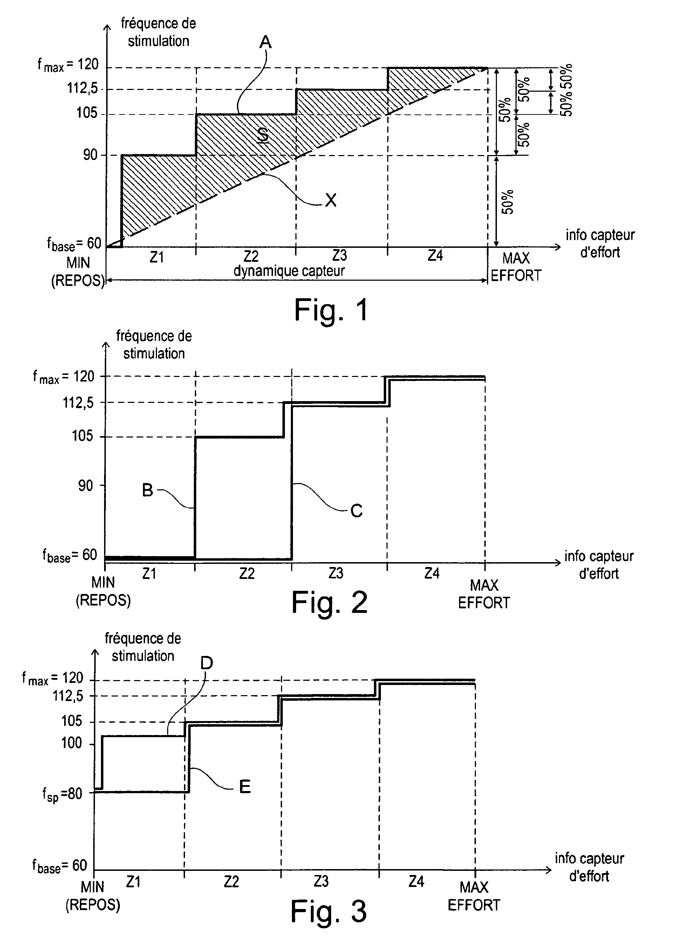

La Figure 1 présente une caractéristique donnant la fréquence de stimulation en fonction de l'information délivrée par le capteur d'effort.

Dans un dispositif classique, cette caractéristique présente une forme telle que celle référencée X, à savoir une caractéristique sensiblement linéaire où la fréquence de stimulation augmente proportionnellement avec le niveau d'effort indiqué par le capteur, depuis la fréquence de base f_{base} jusqu'à la fréquence maximale fₘₐₓ.

L'invention propose, tout en gardant les mêmes bornes extrêmes (fréquence de base f_{base} pour l'effort minimum et fréquence maximale fₘₐₓ pour l'effort maximum), de suivre un autre profil de variation, permettant d'accélérer beaucoup plus la fréquence de stimulation pour les efforts modérés - ces efforts modérés étant chez les insuffisants cardiaques (i) les plus fréquents, car de tels patients exécutent rarement des efforts soutenus et (ii) les plus mal compensés, pour les raisons exposées en introduction (double conséquence de l'effet chronotrope des bêta-bloquants et de l'inadaptation des asservissements des dispositifs actuels).

L'idée de base de l'invention consiste, en cas de détection d'un début d'effort, à calculer une fréquence-cible fonction de l'écart entre la fréquence de base f_{base} (ou la fréquence courante spontanée ou stimulée), d'une part, et la fréquence maximale fₘₐₓ d'autre part, et piloter le stimulateur pour atteindre cette fréquence-cible en un nombre très faible de cycles cardiaques, typiquement 3 à 4 cycles cardiaques.

La fréquence-cible peut en particulier être un pourcentage, par exemple 50% (ou 30%), de la différence entre la fréquence de base (ou la fréquence courante), et la fréquence maximale.

Ce principe est avantageusement appliqué sélectivement sur plusieurs zones distinctes de la dynamique du capteur. Pour une zone donnée; l'écart est alors calculé entre, d'une part, la fréquence-cible de la zone immédiatement inférieure (au lieu de la fréquence de base) et, d'autre part, la fréquence maximale fₘₐₓ.

Plus précisément, comme illustré Figure 1, la dynamique du capteur d'effort est par exemple divisée en quatre zones Z1 à Z4, ces zones étant consécutives sur l'étendue de la dynamique du capteur d'effort.

Dans l'exemple illustré, on a représenté quatre zones d'étendue égale (25% de la dynamique du capteur d'effort pour chacune des zones Z1 à Z4), mais cette caractéristique n'est pas limitative, et il est possible en variante de prévoir une répartition différente des zones successives, avec des zones plus étroites pour les efforts les plus faibles (zone Z1, ou zones Z1 et Z2) de manière à procurer une plus forte accélération dans ces zones correspondant à des efforts modérés, pour lesquels la réactivité doit être maximale.

Si l'on prend l'exemple illustré Figure 1, la caractéristique A donnant, selon l'invention, la fréquence de stimulation en fonction de l'information délivrée par le capteur d'effort est déterminée de la manière suivante.

On considérera tout d'abord le cas d'un patient qui est dès l'origine dans un état stimulé.

Initialement (au repos) la fréquence de stimulation est la fréquence de base f_{base}, par exemple 60 cpm dans l'exemple.

En cas d'effort modéré, le capteur d'effort va délivrer une information comprise dans la zone Z1. Le dispositif va alors calculer une fréquence-cible correspondant par exemple à la moitié de l'intervalle compris entre la fréquence de base f_{base} (60 cpm) et la fréquence maximale programmée fₘₐₓ (par exemple 120 cpm dans l'exemple illustré), soit une fréquence-cible de : 60 + 50%(120-60) = 90 cpm.

Dans le cas où l'effort continue à augmenter, l'information du capteur d'effort se situera dans la zone Z2. Le calcul est effectué de la même façon, mais en prenant à la place de la fréquence de base, la fréquence-cible de la zone Z1 (90 cpm), ce qui donne pour la zone Z2 une nouvelle fréquence-cible de : 90 + 50%(120-90) = 105 cpm.

Le même processus de dichotomie est appliqué à la zone Z3, dont la fréquence-cible sera : 105 + 50%(120-105) = 112,5 cpm.

Quant à la fréquence-cible de la zone Z4, à savoir la zone des efforts les plus intenses, elle sera fixée systématiquement à la valeur de la fréquence maximale, soit 120 cpm dans cet exemple.

Dans le cas d'un effort progressif, croissant depuis une valeur modérée jusqu'à une valeur maximale, c'est-à-dire pour une information de capteur d'effort passant successivement par toutes les zones Z1 à Z4, on obtient ainsi la caractéristique référencée A sur la Figure 1.

On peut constater que pour des efforts modérés l'accélération de la fréquence cardiaque selon le profil de l'invention (caractéristique A) est bien supérieure à ce qu'aurait fourni un asservissement conventionnel (caractéristique X). L'aire S comprise entre les caractéristiques A et X constitue ainsi une "réserve cardiaque" permettant d'augmenter le débit cardiaque du patient, car une partie de l'augmentation du rythme cardiaque va conduire à une augmentation du débit cardiaque.

La Figure 2 illustre le cas d'un patient effectuant un effort brutal, avec une information de capteur d'effort située tout de suite dans la zone Z2, sans passage par la zone Z1.

Pour un tel effort brutal, sans passage en zone Z1, la fréquence-cible appliquée sera calculée selon la règle exposée plus haut, mais en augmentant de 25% le pourcentage de 50% afin d'assurer une fréquence supérieure. Ceci correspond à la caractéristique B.

La même règle peut être appliquée au cas d'un effort encore plus brutal, avec une information de capteur d'effort située tout de suite dans la zone Z3, sans passage en zones Z1 et Z2. Ceci correspond à la caractéristique C. Pour réaliser ces adaptations, l'algorithme examine si, lorsqu'il se trouve dans une zone donnée (Z2 ou Z3), il est déjà passé, ou non, par les zones précédentes (Z1 ou Z2, respectivement).

La Figure 3 est homologue de la Figure 1, mais dans le cas où le patient présente au repos un rythme spontané.

La fréquence spontanée fₛₚ est généralement supérieure à la fréquence de base f_{base}, par exemple fₛₚ = 80 cpm.

Dans ce cas, le calcul de la fréquence-cible est effectué de la manière décrite plus haut à propos de la Figure 1 pour un rythme stimulé, mais en substituant la fréquence spontanée fₛₚ à la fréquence de base f_{base}. L'autre terme du calcul de la différence reste la fréquence maximale fₘₐₓ, ce qui donne avec les valeurs de cet exemple une valeur de fréquence-cible de : 80 + 50%(120-80) = 100 cpm.

En généralisant, ceci signifie que pour le calcul de la fréquence-cible le dispositif considère la plus rapide de la fréquence de base f_{base} et de la fréquence spontanée courante fₛₚ. Ceci permet d'assurer que l'incrément de fréquence calculé par l'algorithme et appliqué par le dispositif sera dans tous les cas suffisamment significatif pour soutenir l'effort. Ainsi, dans l'exemple le résultat est une fréquence-cible de 100 cpm (soit un pas de fréquence de 20 cpm supplémentaires) en présence d'un rythme spontané, au lieu de 90 cpm dans le cas de la Figure 1.

Cette situation est illustrée par la caractéristique D de la Figure 3. Comme précédemment, dans le cas d'un effort brutal donnant une information de capteur d'effort située directement dans la zone Z2 sans passer par la zone Z1, la caractéristique serait celle illustrée en E sur la Figure 3.

## Revendications

1. Un dispositif médical implantable actif du type prothèse cardiaque de stimulation, resynchronisation et/ou défibrillation, pourvue de moyens CRT de stimulation synchronisée pour le traitement de l'insuffisance cardiaque, avec :
- des moyens pour déterminer une valeur courante de fréquence spontanée (fₛₚ) du patient en présence d'un rythme sinusal ;
- un capteur d'effort, délivrant un signal représentatif du niveau courant d'effort du patient ;
- des moyens pour détecter le début d'un effort à partir du signal délivré par le capteur ;
- des moyens pour calculer une fréquence de stimulation asservie à l'effort, cette fréquence de stimulation pouvant varier entre une fréquence de base (f_{base}) et une fréquence maximale (fₘₐₓ) en fonction du signal délivré par le capteur ; et
- des moyens pour délivrer sélectivement des impulsions de stimulation à ladite fréquence de stimulation calculée,
dispositif **caractérisé en ce que** les moyens CRT de stimulation synchronisée comprennent en outre des moyens d'incrémentation accélérée de la fréquence de stimulation, aptes à exécuter des commandes de :
- détermination d'une fréquence-cible fonction de l'écart entre :
• d'une part, la plus élevée d'entre la fréquence de base (f_{base}) et, le cas échéant, la fréquence spontanée (fₛₚ), et
• d'autre part la fréquence maximale (f_{maX}) ; et
- augmentation directe, sur détection d'un début d'effort, de la fréquence de stimulation depuis la valeur initiale courante de la fréquence de stimulation, ou de la fréquence spontanée, jusqu'à ladite fréquence-cible en un laps de temps prédéterminé, ou sur un nombre de cycles cardiaques prédéterminé, permettant d'atteindre ladite fréquence-cible en au plus quatre cycles cardiaques.

2. Le dispositif médical CRT de la revendication 1, comprenant en outre des moyens pour définir une pluralité de zones d'effort consécutives (Z1-Z4) sur l'étendue de la dynamique du signal délivré par le capteur d'effort, et dans lequel les moyens d'incrémentation accélérée de la fréquence de stimulation sont aptes à exécuter distinctement pour chaque zone lesdites commandes de détermination de fréquence-cible et d'augmentation directe de la fréquence de stimulation jusqu'à cette fréquence-cible, la fréquence-cible d'une zone étant fonction de l'écart entre :
• d'une part, la fréquence-cible de la zone immédiatement précédente, et
• d'autre part la fréquence maximale (fₘₐₓ).

3. Le dispositif médical CRT de la revendication 2, dans lequel lesdites zones d'effort consécutives (Z1-Z4) sont définies par une division égale de l'étendue de la dynamique du signal délivré par le capteur d'effort.

4. Le dispositif médical CRT de la revendication 2, comprenant en outre des moyens de mise à jour des valeurs extrêmes de la dynamique du signal délivré par le capteur d'effort, et de redéfinition de ladite pluralité de zones d'effort consécutives (Z1-Z4).

5. Le dispositif médical CRT de la revendication 1, dans lequel la fréquence-cible est déterminée par un pourcentage de l'écart entre d'une part la plus élevée d'entre la fréquence de base (f_{base}) et, le cas échéant, la fréquence spontanée (fₛₚ), et d'autre part la fréquence maximale (fₘₐₓ).

6. Le dispositif médical CRT de la revendication 4, dans lequel ledit pourcentage est 50%.

## Claims

1. Active implantable medical device of the cardiac prosthesis type for stimulation, resynchronization and/or defibrillation, provided with synchronized stimulation CRT means for the treatment of cardiac insufficiency, with:
- means for determining a current spontaneous frequency value (fₛₚ) of the patient in the presence of a sinusoidal rhythm;
- an effort sensor, delivering a signal representative of the current level of effort of the patient;
- means for detecting the start of an effort from the signal delivered by the sensor;
- means for calculating a stimulation frequency slaved to the effort, this stimulation frequency being able to vary between a base frequency (f_{base}) and a maximum frequency (fₘₐₓ) as a function of the signal delivered by the sensor; and
- means for selectively delivering stimulation pulses at said calculated stimulation frequency,
the device being **characterized in that** the synchronized stimulation CRT means also comprise stimulation frequency accelerated incrementation means, suitable for executing commands for:
- determining a target frequency that is a function of the difference between:
• on the one hand, the highest of the base frequency (f_{base}) and, where appropriate, the spontaneous frequency (fₛₚ), and
• on the other hand, the maximum frequency (fₘₐₓ); and
- directly increasing, on detection of a start of effort, the stimulation frequency from the current initial value of the stimulation frequency, or of the spontaneous frequency, up to said target frequency in a predetermined time period, or over a predetermined number of cardiac cycles, making it possible to reach said target frequency in no more than four cardiac cycles.

2. CRT medical device of Claim 1, also comprising means for defining a plurality of consecutive effort regions (Z1-Z4) over the extent of the dynamic range of the signal delivered by the effort sensor, and in which the stimulation frequency accelerated incrementation means are suitable for executing, distinctly for each region, said commands for determining target frequency and for directly increasing the stimulation frequency up to this target frequency, the target frequency of a region being a function of the difference between:
• on the one hand, the target frequency of the immediately preceding region, and
• on the other hand, the maximum frequency (fₘₐₓ).

3. CRT medical device of Claim 2, in which said consecutive effort regions (Z1-Z4) are defined by an equal division of the extent of the dynamic range of the signal delivered by the effort sensor.

4. CRT medical device of Claim 2, also comprising means for updating the extreme values of the dynamic range of the signal delivered by the effort sensor, and for redefining said plurality of consecutive effort regions (Z1-Z4).

5. CRT medical device of Claim 1, in which the target frequency is determined by a percentage of the difference between, on the one hand, the highest of the base frequency (f_{base}) and, where appropriate, the spontaneous frequency (fₛₚ), and, on the other hand, the maximum frequency (fₘₐₓ).

6. CRT medical device of Claim 4, in which said percentage is 50%.

## Patentansprüche

1. Aktive implantierbare medizinische Vorrichtung des Typs Resynchronisations-, Defibrillations- und/oder Stimulations-Herzprothese, die mit CRT-Mitteln für die synchronisierte Stimulation zur Behandlung von Herzinsuffizienz versehen ist, mit:
- Mitteln, um einen momentanen spontanen Frequenzwert (fₛₚ) des Patienten in Gegenwart eines Sinus-Rhythmus zu bestimmen;
- einem Anstrengungssensor, der ein Signal liefert, das den momentanen Anstrengungsgrad des Patienten repräsentiert;
- Mitteln, um den Beginn einer Anstrengung anhand des von dem Sensor gelieferten Signals zu detektieren;
- Mitteln, um eine auf die Anstrengung geregelte Stimulationsfrequenz zu berechnen, wobei diese Stimulationsfrequenz als Funktion des von dem Sensor gelieferten Signals zwischen einer Grundfrequenz (f_{base}) und einer Maximalfrequenz (fₘₐₓ) variieren kann; und
- Mitteln, um wahlweise Stimulationsimpulse mit der berechneten Stimulationsfrequenz auszugeben,
wobei die Vorrichtung **dadurch gekennzeichnet ist, dass** die CRT-Mittel für synchronisierte Stimulation außerdem Mittel für beschleunigte Inkrementierung der Stimulationsfrequenz umfassen, die dafür ausgelegt sind, folgende Befehle auszuführen:
- Bestimmen einer Sollfrequenz als Funktion des Abstands zwischen:
- einerseits dem höchsten Wert aus der Menge, die die Grundfrequenz (f_{base}) und gegebenenfalls die spontane Frequenz (fₛₚ) umfasst, und
- andererseits der Maximalfrequenz (fₘₐₓ); und
- direktes Erhöhen der Stimulationsfrequenz bei Detektion eines Anstrengungsbeginns ausgehend von dem momentanen Anfangswert der Stimulationsfrequenz oder der spontanen Frequenz bis zu der Sollfrequenz in einem vorgegebenen Zeitintervall oder über eine vorgegebene Anzahl von Herzzyklen hinweg, was ermöglicht, die Sollfrequenz in höchstens vier Herzzyklen zu erreichen.

2. Medizinische CRT-Vorrichtung nach Anspruch 1, die außerdem Mittel umfasst, um mehrere aufeinanderfolgende Anstrengungszonen (Z1-Z4) über den dynamischen Bereich des von dem Anstrengungssensor gelieferten Signals zu definieren, wobei die Mittel für beschleunigte Inkrementierung der Stimulationsfrequenz dafür ausgelegt sind, für jede Zone die Befehle des Bestimmens der Sollfrequenz und des direkten Erhöhens der Stimulationsfrequenz bis zu dieser Sollfrequenz unabhängig auszuführen, wobei die Sollfrequenz einer Zone eine Funktion des Abstands ist zwischen:
- einerseits der Sollfrequenz der unmittelbar vorhergehenden Zone und
- andererseits der Maximalfrequenz (fₘₐₓ).

3. Medizinische CRT-Vorrichtung nach Anspruch 2, wobei die aufeinanderfolgenden Anstrengungszonen (Z1-Z4) durch eine gleiche Unterteilung des dynamischen Bereichs des von dem Anstrengungssensor gelieferten Signals definiert sind.

4. Medizinische CRT-Vorrichtung nach Anspruch 2, die außerdem Mittel zum Aktualisieren der Extremwerte der Dynamik des von dem Anstrengungssensor gelieferten Signals und zum erneuten Definieren der mehreren aufeinanderfolgenden Anstrengungszonen (Z1-Z4) umfasst.

5. Medizinische CRT-Vorrichtung nach Anspruch 1, wobei die Sollfrequenz für einen Prozentsatz des Abstands einerseits zwischen dem höchsten Wert aus der Menge, die die Grundfrequenz (f_{base}) und gegebenenfalls die spontane Frequenz (fₛₚ) umfasst, und andererseits der Maximalfrequenz (fₘₐₓ) bestimmt wird.

6. Medizinische CRT-Vorrichtung nach Anspruch 4, wobei der Prozentsatz 50 % beträgt.
